(19) Europäisches Patentamt
European Patent Office
Office européen des brevets

(11) Veröffentlichungsnummer : **0 084 671**
**B1**

(12) # EUROPÄISCHE PATENTSCHRIFT

(45) Veröffentlichungstag der Patentschrift : **05.03.86**

(21) Anmeldenummer : **82111946.8**

(22) Anmeldetag : **23.12.82**

(51) Int. Cl.⁴ : **C 07 C103/37**, C 07 C103/42, C 07 C103/38, C 07 C125/065, C 07 C127/19, C 07 C155/03, C 07 D295/20, C 07 C 97/10, C 07 C 91/40, C 07 C 87/48, C 07 C 93/14

(54) **Anilinderivate, Verfahren zu ihrer Herstellung und ihre Verwendung zur Bekämpfung unerwünschten Pflanzenwuchses sowie Zwischenprodukte für ihre Herstellung.**

(30) Priorität : **27.01.82 DE 3202624**

(43) Veröffentlichungstag der Anmeldung : **03.08.83 Patentblatt 83/31**

(45) Bekanntmachung des Hinweises auf die Patenterteilung : **05.03.86 Patentblatt 86/10**

(84) Benannte Vertragsstaaten : **BE DE FR GB IT NL**

(56) Entgegenhaltungen :
**EP-A- 0 041 145**
**DE-A- 2 220 383**
**SU-A- 174 181**

(73) Patentinhaber : **BASF Aktiengesellschaft**
**Carl-Bosch-Strasse 38**
**D-6700 Ludwigshafen (DE)**

(72) Erfinder : **Schirmer, Ulrich, Dr.**
**Berghalde 79**
**D-6900 Heidelberg (DE)**
Erfinder : **Plath, Peter, Dr.**
**Berner Weg 24**
**D-6700 Ludwigshafen (DE)**
Erfinder : **Wuerzer, Bruno, Dr.**
**Ruedigerstrasse 13**
**D-6701 Otterstadt (DE)**

EP 0 084 671 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Anilinderivate, Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

Es ist bekannt, daß N-Methoxy-N-methyl-N'-phenyl-harnstoffe herbizid wirksam sind. Weiterhin ist bekannt, daß auch N-(Phenylalkyl-phenyl)-harnstoffderivate herbizid wirksam sind (EP-A-41 145).

Es wurde gefunden, daß Anilinderivate der Formel

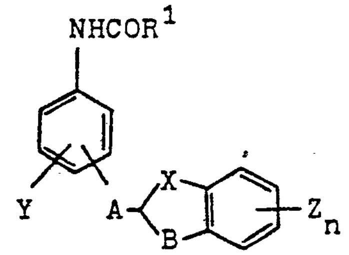

(I)

in der

$R^1$ Alkoxy, Alkenoxy, Alkinoxy, Alkylthio oder gegebenenfalls durch Halogen oder Alkoxy mit 1 bis 5 kohlenstoffatomen substituiertes Alkyl mit jeweils bis zu 4 Kohlenstoffatomen oder Cycloalkyl mit 3 bis 6 Kohlensfoffatomen oder den Rest

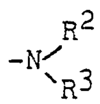

wobei $R^2$ und $R^3$ unabhängig voneinander Wasserstoff, Alkyl, Alkenyl, Alkinyl oder Alkoxy mit jeweils bis zu 4 Kohlenstoffatomen oder Cycloalkyl oder Cycloalkoxy mit 3 bis 6 Kohlenstoffatomen bedeuten oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

X Methylen, —CH(OH)— oder —CO—,

Y Wasserstoff, Halogen, Methyl, Methoxy oder Trifluormethyl,

A eine gegebenenfalls durch Alkyl mit 1 bis 5 Kohlenstoffatomen substituierte Alkylenkette mit 1 bis 5 Kohlenstoffatomen,

B gegebenenfalls durch Alkyl mit 1 bis 3 Kohlenstoffatomen substituiertes Methylen oder Ethylen,

Z Wasserstoff, Halogen, Alkyl, Alkoxy, Halogenalkyl oder Halogenalkoxy mit jeweils bis zu 6 Kohlenstoffatomen, Phenyl oder Phenoxy und

n die Zahlen 1, 2 oder 3 bedeuten,

eine herbizide Wirkung haben und für eine Reihe von Kulturpflanzen eine hohes Maß an Verträglichkeit besitzen,

Die Substituenten in Formel I können die folgenden Bedeutungen haben :

$R^1$ bedeutet Alkoxy, Alkenoxy, Alkinoxy, Alkylthio oder gegebenenfalls durch Halogen oder Alkoxy mit 1 bis 4 Kohlenstoffatomen substituiertes Alkyl mit jeweils bis zu 5 Kohlenstoffatomen, beispielsweise Methoxy, Ethoxy, n-Propoxy, Isopropoxy, sec.-Butoxy, tert.-Butoxy, n-Pentoxy, Propargyloxy, Allyloxy, 1-Methyl-propgargyloxy, Methylthio, Ethylthio, n-Propylthio, sec.-Butylthio, tert.-Butylthio, Methyl, Ethyl, n-Propyl, n-Butyl, sec.-Butyl, tert.-Butyl, Isobutyl, n-Pentyl, Chlormethyl, 1,1-Dichlorethyl, Dichlormethyl, Trichlormethyl, Methoxymethyl, 1-Methoxyethyl, 2-Methoxyethyl, Cycloalkyl mit 3 bis 6 Kohlenstoffatomen, beispielsweise Cyclopropyl, Cyclopentyl, Cyclohexyl, Cycloalkoxy mit 3 bis 6 Kohlenstoffatomen, beispielsweise Cyclohexoxy, oder den Rest

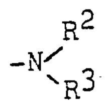

wobei $R^2$ und $R^3$ unabhängig voneinander Wasserstoff, Alkyl, Alkenyl, Alkinyl oder Alkoxy mit jeweils bis zu 4 Kohlenstoffatomen, beispielsweise Methyl, Ethyl, Isopropyl, n-Butyl, sec.-Butyl, Isobutyl, tert.-Butyl, Allyl, 1,1-Dimethylpropargyl, Methoxy, Ethoxy, oder Cycloalkyl mit 3 bis 6 Kohlenstoffatomen, beispielsweise Cyclopropyl, Cyclopentyl, Cyclohexyl, oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen, beispielsweise Trimethylen, Tetramethylen, 1,4-Dimethyltetramethylen, Pentamethylen, Hexamethylen, 3-Oxapentamethylen, 1-Oxapentamethylen, 1-Oxa-tetramethylen, bedeuten.

Y bedeutet Wasserstoff, Halogen, beispielsweise Fluor, Chlor, Brom, Methyl, Methoxy oder Trifluormethyl.

A steht in meta- oder para-Stellung zu der Gruppe —NH—CO—$R^1$ und bedeutet eine Alkylenkette mit

1 bis 5 Kohlenstoffatomen, die durch Alkyl mit 1 bis 5 Kohlenstoffatomen, insbesondere durch Methyl, substituiert sein kann, beispielsweise $—CH_2—$, $—CH(CH_3)—$, $—(CH_2)_3—$, $—(CH_2)_2—$, $—CH_2CH(CH_3)CH_2$, $—(CH_2)_5—$ oder $—CH_2CH(CH_3)(CH_2)_3—$.

B bedeutet gegebenenfalls durch Alkyl mit 1 bis 3 Kohlenstoffatomen, insbesondere durch Methyl, substituiertes Methylen oder Dimethylen, beispielsweise $—CH_2—$, $—CH(CH_3)—$, $—(CH_2)_2—$, $—CH(CH_3)CH_2—$ oder $—CH_2CH(CH_3)—$.

Z bedeutet Wasserstoff, Halogen, beispielsweise Fluor, Chlor, Brom, Alkyl, Alkoxy, Halogenalkyl oder Halogenalkoxy mit jeweils bis zu 6 Kohlenstoffatomen, beispielsweise Methyl, Ethyl, Isopropyl, tert.-Butyl, n-Hexyl, Methoxy, Ethoxy, Isopropoxy, tert.-Butoxy, n-Hexoxy, Trifluormethyl, 1,1,2-Trifluor-2-chlorethoxy, Difluormethoxy, Phenyl oder Phenoxy. Z steht in ortho-, meta- oder para-Stellung zu X ; n ist vorzugsweise 1, kann jedoch auch 2 oder 3 bedeuten, insbesondere, wenn Z für Fluor, Chlor, Brom oder Methyl steht.

Bevorzugte Verbindungen der Formel I sind solche, bei denen $R^1$ den Rest

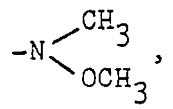

Y Wasserstoff oder Halogen, insbesondere Chlor, A Methylen in para- oder meta-Stellung zu der Gruppe $—NHCOR^1$, B Methylen oder Dimethylen, X Methylen, Z Halogen, insbesondere Chlor, oder Alkyl mit bis zu 4 Kohlenstoffatomen, insbesondere Methyl oder Methoxy, und n 1 bedeuten.

Man erhält die Anilinderivate der Formel I durch Umsetzung von Aminen der Formel

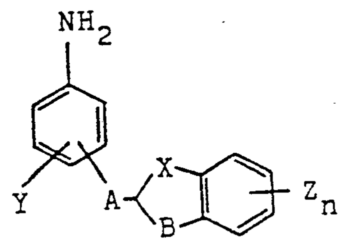

(II)

in der A, Y, X, B, Z und n die obengenannten Bedeutungen haben, mit Verbindungen der Formel

$$R^1—CO—X \quad (III)$$

in der $R^1$ die obengenannten Bedeutungen hat und X eine Abgangsgruppe, vorzugsweise Halogen, wie Chlor oder Brom, oder $R^1—CO—O—$ bedeutet.

Die Umsetzung wird in einem inerten organischen Lösungsmittel durchgeführt. Geeignet sind Ether, wie Tetrahydrofuran, Dimethoxyethan, Diethylether, Methyl-tert.-butylether, Ester wie Essigsäureethylester, gegebenenfalls chlorierte aliphatische oder aromatische Kohlenwasserstoffe, wie Toluol, Dichlormethan, Pyridin. Auch Gemische dieser Lösungsmittel können verwendet werden. Die Menge an Lösungsmittel, bezogen auf Amin der Formel II beträgt 100 bis 5 000 Gew.-%.

Zweckmäßigerweise wird die Umsetzung in Gegenwart eines Säureakzeptors durchgeführt. In Betracht kommen Alkalihydroxide, Alkalicarbonate, Alkalihydrogencarbonate, Erdalkalihydroxide, Erdalkalicarbonate, Erdalkalihydrogencarbonate, Erdalkalioxide oder Amine, beispielsweise Natriumhydrogencarbonat, Kaliumcarbonat, Triethylamin, Pyridin, N,N-Dimethylanilin, N,N-Dimethyl-N-cyclohexylamin, Chinolin. Die Menge an Säureakzeptor beträgt 1 bis 4 Mol, bezogen auf 1 Mol Verbindung der Formel III.

Die Ausgangsstoffe der Formeln II und III werden bevorzugt in äquimolarem Verhältnis miteinander umgesetzt. Die Reaktionstemperatur liegt zwischen 20 und 80 °C, vorzugsweise zwischen 20 und 30 °C.

Anilinderivate der Formel I, bei denen $R^1$ Alkoxy oder Alkylthio oder den Rest

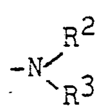

und X Methyl oder —CO— bedeuten und A, B, Y, Z und n die obengenannten Bedeutungen haben, erhält man auch durch Umsetzung von Isocyanaten der Formel

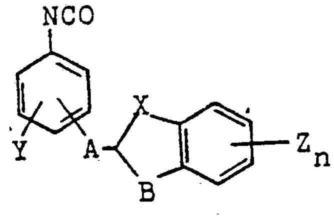

(IV)

in der Y, A, B, X, Z und n die vorstehend genannten Bedeutungen haben, mit einer Verbindung der Formel $R^1H$, in der $R^1$ die vorstehend genannten Bedeutungen hat, bei einer Temperatur zwischen 0 und 150, vorzugsweise zwischen 10 und 50 °C.

Die Umsetzung wird in einem unter den Reaktionsbedingungen inerten Lösungsmittel durchgeführt. Geeignet sind Ether, wie Diethylether, Methyl-tert.-butyl-ether, Tetrahydrofuran, Ester, wie Essigsäureethylester, gegebenenfalls chlorierte aliphatische oder aromatische Kohlenwasserstoffe, wie Ligroin, Toluol, Cyclohexan, Benzin, Dichlormethan, Chloroform, Chlorbenzol, o-, m-, p-Dichlorbenzol, Nitrobenzol, Ketone, wie Aceton, Nitrile, wie Acetonitril oder Dimethylformamid. Auch Gemische dieser Lösungsmittel können verwendet werden.

Die Umsetzung der Isocyanate der Formel IV mit Alkoholen, Mercaptanen oder Aminen der Formel $R^1H$ kann gegebenenfalls durch Zugabe eines für Isocyanatreaktionen gebräuchlichen Katalysators beschleunigt werden, z. B. durch tert.-Amine, wie Triethylamin, 1,4-Diaza-bicyclo [2,2,2] octan, Stickstoffheterocyclen, wie Pyridin oder 1,2-Dimethylimidazol oder organische Zinnverbindungen, wie Dibutylzinndiacetat, Dimethylzinndichlorid.

Zur Herstellung des Isocyanate gemäß Formel IV setzt man die Amine der Formel III mit Phosgen um (Liebigs Ann. Chem. *562*, 75ff, (1949)).

Die Amine der Formel II sind neu. Sie werden nach bekannten Verfahren durch Aldolkondensation und anschließende Hydrierung erhalten. Man setzt beispielsweise gegebenenfalls substituierte alpha-Indanone oder alpha-Tetralone mit Nitrobenzaldehyden zu Nitrochalkonen um (R. J. Murray, N. A. Cromwell, J. Org. Chem. *41*, 3540-3545 (1976)).

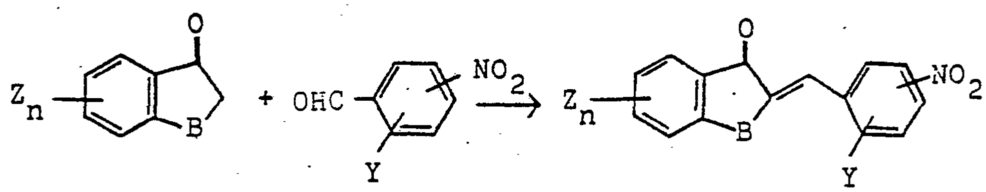

und hydriert die so erhaltenen Nitrochalkone in an sich bekannter Weise in einem Essigsäure/Schwefelsäure-Gemisch (wenn X = Methylen) bzw. in einen organischen Lösungsmittel, wie Methanol oder Tetrahydrofuran (wenn X = —CO— oder —CH(OH)—), Essigsäure in Gegenwart von Palladium auf Kohle oder Raney-Nickel bei Temperaturen zwischen 20 und 150 °C, vorzugsweise zwischen 20 und 80 °C, gegebenenfalls unter Druck. B, Y, Z und n haben dabei die obengenannten Bedeutungen.

Amine der Formel II, bei denen A $-CH_2CH(CH_3)CH_2-$ oder $-(CH_2)_3-$ bedeutet, werden dadurch erhalten, daß man gegebenenfalls substituierte alpha-Tetralone oder alpha-Indanone mit Nitrozimtaldehyden nach folgendem Schema kondensiert

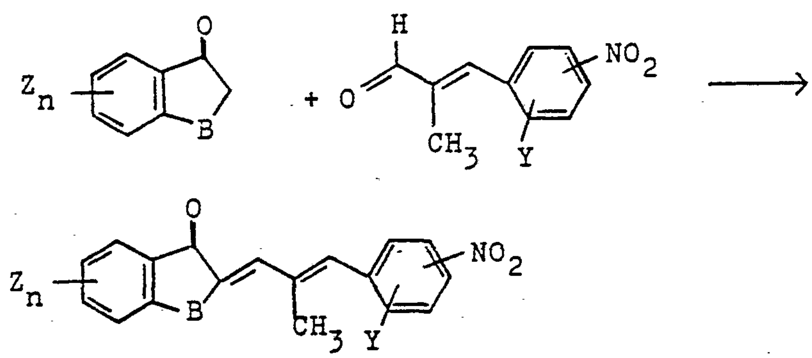

und anschließend hydriert.

Die Wasserstoffaufnahme bei der Hydrierung der vorgenannten Nitrophenylchalkone erfolgt stufenweise und kann beim Arbeiten in Tetrahydrofuran entweder nach Erreichen der gesättigten Anilinoketon-Stufe (X = —CO—) abgebrochen werden oder bis zur Anilinocarbinolstufe (X = —CH(OH)—) weitergeführt werden, bzw. beim Arbeiten in Essigsäure/Schwefelsaure-Gemisch direkt bis zur Anilino-Kohlenwasserstoffstufe (X = $-CH_2-$) durchgeführt werden.

Die Hydrierung erfolgt in an sich bekannter Weise in Gegenwart von Raney-Nickel oder vorzugsweise von Palladium auf Aktivkohle. In diesem Fall gelingt die Hydrierung zum gesättigten Anilinoketon (X = —CO—) bereits bei Temperaturen von 0 bis 30 °C, vorzugsweise bei 25 °C, und bei einem Druck von 1,01 bis 20 bar, bevorzugt bei 1,1 bar. Verlängerung der Reaktionsdauer, Erhöhung der Hydriertemperatur und/oder Druckerhöhung bis zu 50 bar führt zur Reduktion der Ketogruppe zur Carbinolgruppe (X = —CH(OH)—) (Houben-Weyl, Methoden der Org. Chemie, Bd. 4/1c, S. 13ff, Georg-Thieme-Verlag, Stuttgart, 1980).

Die folgenden Beispiele veranschaulichen die Synthese der Amine der Formel II :

Beispiel A

114,8 g 4-Nitrobenzaldehyd, 100 g Indanon und 10 g Borsäure wurden unter Wasserauskreisung in 500 ml Xylol 12 Stunden lang gekocht, die noch heiße Lösung in Toluol eingerührt und nach dem Erkalten abgesaugt. Man erhielt 115 g (Ausbeute : 57 %) 4'-Nitrobenzylidenindanon.

113 g 4'-Nitrobenzylidenindanon wurden in 42 g Schwefelsäure und 200 ml Eisessig gelöst, mit 10 g Palladium/Aktivkohle (10 %ig) versetzt und bei einem Druck von 1,1 bar bei 70 °C bis zur Aufnahme von 50 l Wasserstoff hydriert. Danach wurde filtriert, eingeengt mit 10 %iger Natronlauge alkalisch gemacht und mit Diethylether ausgeschüttelt. Nach Trocknen der organischen Phase über Natriumsulfat, Filtrieren und Einengen erhielt man 68,3 g (Ausbeute 72 %) Amin der Formel

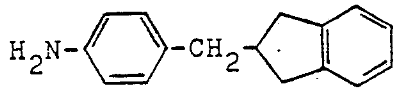

vom Schmelzpunkt 39 bis 41 °C.

Beispiel B

50 g 4'-Nitrophenylbenzylidenindanon wurden in 1 000 ml Tetrahydronfuran gelöst und bei 20 bis 25 °C in Gegenwart von 4 g 10 %iger Palladium-Tierkohle und einem Druck von 1,1 bar bis zur Aufnahme von 17,8 l Wasserstoff hydriert. Nach dem Trocknen über Natriumsulfat wurde filtriert und eingeengt. Der verbliebene Rückstand wurde mit Petrolether vermischt und abgesaugt. Man erhielt 38 g (Ausbeute : 86 %) Amin der Formel

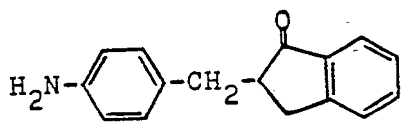

vom Schmelzpunkt 123 bis 126 °C.

Entsprechend können folgende Amine der Formel II hergestellt werden :

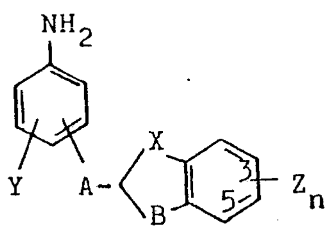

| Y | A | X | B | $Z_n$ | Kp/Fp [°C] |
|---|---|---|---|---|---|
| H | $3-CH_2-$ | $-CH_2-$ | $-CH_2-$ | H | Fp 71- 73 |
| H | $3-CH_2-$ | $-CH(OH)-$ | $-CH_2-$ | H | |
| H | $3-CH_2-$ | $-CO-$ | $-CH_2-$ | H | |
| H | $3-CH_2-$ | $-CH_2-$ | $-CH_2-CH_2-$ | H | $Kp_{0,3}$ 165-168 |
| H | $3-CH_2-$ | $-CO-$ | $-CH_2-CH_2-$ | H | Öl |
| H | $3-CH_2-$ | $-CH(OH)-$ | $-CH_2-CH_2-$ | H | |
| H | $4-CH_2-$ | $-CH(OH)-$ | $-CH_2-$ | H | |
| H | $4-CH_2-$ | $-CH_2-$ | $-CH_2-CH_2-$ | H | $Kp_{0,4}$ 171-172 |
| H | $4-CH_2-$ | $-CO-$ | $-CH_2-CH_2-$ | H | |
| H | $4-CH_2-$ | $-CH(OH)-$ | $-CH_2-CH_2-$ | H | |
| $3-CH_3$ | $4-CH_2-$ | $-CH_2-$ | $-CH_2-$ | H | |
| 3-Cl | $4-CH_2-$ | $-CO-$ | $-CH_2-$ | H | |
| $4-CH_3$ | $3-CH_2-$ | $-CH_2-$ | $-CH_2-CH_2-$ | H | |
| 4-Cl | $3-CH_2-$ | $-CO-$ | $-CH_2-CH_2-$ | H | |

(Fortsetzung)

| Y | A | X | B | $Z_n$ | Kp/Fp [°C] |
|---|---|---|---|---|---|
| H | $4-(CH_2)_3-$ | $-CH_2-$ | $-CH_2-$ | H | |
| H | $3-(CH_2)_3-$ | $-CH_2-$ | $-CH_2-CH_2-$ | H | |
| H | $3-CH_2CH(CH_3)CH_2-$ | $-CH_2-$ | $-CH_2-CH_2-$ | H | |
| H | $4-CH_2CH(CH_3)CH_2-$ | $-CH_2-$ | $-CH_2-$ | H | |
| H | $4-CH_2-$ | -CO- | $-CH(CH_3)-$ | 3-Cl | Öl |
| H | $3-CH_2-$ | $-CH_2-$ | $-CH_2-CH_2-$ | $4-OCH_3$ | |
| H | $4-CH_2-$ | $-CH_2-$ | $-CH_2-$ | $4-OCH_3$ | Öl |
| H | $4-CH_2-$ | $-CH_2-$ | $-CH_2-$ | $3-CH_3$ | Öl |
| H | $4-CH_2-$ | -CO- | $-CH_2-$ | $3-CH_3$ | |
| H | $3-CH_2-$ | $-CH_2-$ | $-CH_2-$ | $4-CH_3$ | Öl |
| H | $3-CH_2-$ | $-CH_2-$ | $-CH_2-CH_2-$ | $4-CH_3$ | |
| H | $4-CH_2-$ | $-CH_2-$ | $-CH_2-$ | $3-t-C_4H_9$ | |
| H | $4-CH_2-$ | $-CH_2-$ | $-CH_2-$ | 3-F | Öl |
| H | $4-CH_2-$ | -CO- | $-CH_2-$ | 3-Br | |
| H | $4-CH_2-$ | $-CH_2-$ | $-CH_2-$ | $3,4-(CH_3)_2$ | |
| H | $4-CH_2-$ | $-CH_2-$ | $-CH_2-$ | $2,4-(CH_3)_2$ | |
| H | $3-CH_2-$ | $-CH_2-$ | $-CH_2-$ | $2,4-(CH_3)_2$ | |
| H | $4-CH_2-$ | $-CH_2-$ | $-CH_2-$ | $4-i-C_3H_7$ | |
| H | $4-CH_2-$ | $-CH_2-$ | $-CH_2-$ | $3,4-(OCH_3)_2$ | |
| H | $4-CH_2-$ | $-CH_2-$ | $-CH_2-$ | 4-Phenyl | |
| H | $4-CH_2-$ | $-CH_2-$ | $-CH_2-$ | 4-Phenoxy | |
| H | $4-CH_2-$ | $-CH_2-$ | $-CH_2-$ | $2,5-(CH_2)_3$ | |
| H | $3-CH_2-$ | $-CH_2-$ | $-CH_2CH(CH_3)-$ | H | |
| H | $4-CH_2-$ | -CO- | $-CH_2-$ | 3-Cl | |
| H | $4-CH_2-$ | -CO- | $-CH_2-$ | 4-Cl | |
| $3-OCH_3$ | $4-CH_2-$ | $-CH_2-$ | $-CH_2-$ | H | |
| $3-CF_3$ | $4-CH_2-$ | $-CH_2-$ | $-CH_2-$ | H | |
| H | $4-CH_2-$ | $-CH_2-$ | $-CH_2-$ | $3-CF_3$ | |
| H | $4-CH_2-$ | $-CH_2-$ | $-CH_2-$ | $3-OCHF_2$ | |
| H | $4-CH_2-$ | $-CH_2-$ | $-CH_2-$ | 3-Cl | Öl |
| H | $3-CH_2-$ | $-CH_2-$ | $-CH_2-$ | $3-OCH_3$ | |
| H | $4-CH_2-$ | -CH(OH)- | $-CH_2-$ | 4-Cl | |
| H | $4-CH_2-$ | -CH(OH)- | $-CH_2-$ | 4-F | |
| H | $4-CH_2-$ | -CO- | $-CH_2-$ | 4-F | |
| H | $3-CH_2$ | -CO- | $-CH_2CH_2-$ | 3-Cl | Öl |
| H | $4-CH_2-$ | -CO- | $-CH_2CH_2-$ | 3-Cl | Öl |
| H | $3-CH_2-$ | $-CH_2-$ | $-CH_2-$ | $4-OCH_3$ | Öl |
| H | $3-CH_2-$ | $-CH_2$ | $-CH_2-$ | 4-F | Öl |

(Fortsetzung)

| Y | A | X | B | $Z_n$ | Kp/Fp [°C] |
|---|---|---|---|---|---|
| H | $3-CH_2-$ | $-CH_2-$ | $-CH(CH_3)-$ | H | Öl |
| H | $4-CH_2-$ | $-CH_2-$ | $-CH(CH_3)-$ | H | Öl |
| H | $3-CH_2-$ | $-CH_2-$ | $-C(CH_3)_2-$ | H | Öl |
| H | $4-CH_2-$ | $-CH_2-$ | $-C(CH_3)_2$ | H | Öl |

Folgendes Beispiel erläutert die Herstellung der Anilin-derivate der Formel I

Beispiel 1

15 g des nach Beispiel A erhaltenen Amins wurden in 150 ml Tetrahydrofuran gelöst, mit 7,6 g Natriumhydrogencarbonat versetzt und unter gutem Rühren bei 20 bis 25 °C mit 8,6 g N-Methoxy-N-methylcarbaminsäurechlorid zur Reaktion gebracht. Nach 12 stündigem Nachrühren wurde filtriert, eingeengt, mit Petrolether verrieben und abgesaugt. Man erhält 18,6 g (Ausbeute 89 %) Anilinderivat der Formel

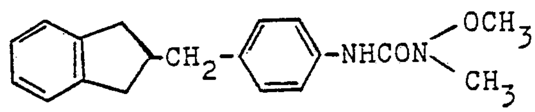

vom Schmelzpunkt 80 bis 82 °C.

Entsprechend können folgende Verbindungen der Formel I hergestellt werden :

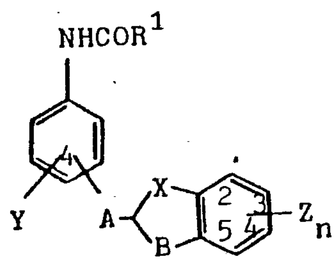

| Nr. | $R^1$ | Y | A | X | B | $Z_n$ | Fp [°C. |
|---|---|---|---|---|---|---|---|
| 2 | $NH-CH_3$ | H | $4-CH_2-$ | $-CH_2-$ | $-CH_2-$ | H | 186-188 |
| 3 | $N(CH_3)_2$ | H | $4-CH_2-$ | $-CH_2-$ | $-CH_2-$ | H | 123-125 |
| 4 | $N(OC_2H_5)CH_3$ | H | $4-CH_2-$ | $-CH_2-$ | $-CH_2-$ | H | 64- 66 |
| 5 | $N(OC_2H_5)C_2H_5$ | H | $4-CH_2-$ | $-CH_2-$ | $-CH_2-$ | H | |
| 6 | 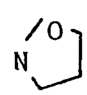 | H | $4-CH_2-$ | $-CH_2-$ | $-CH_2-$ | H | |
| 7 | 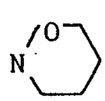 | H | $4-CH_2-$ | $-CH_2-$ | $-CH_2-$ | H | |
| 8 | $C_2H_5$ | H | $4-CH_2-$ | $-CH_2-$ | $-CH_2-$ | H | 117-118 |
| 9 | $SCH_3$ | H | $4-CH_2-$ | $-CH_2-$ | $-CH_2-$ | H | |

(Fortsetzung)

| Nr. | $R^1$ | Y | A | X | B | $Z_n$ | Fp [°C] |
|---|---|---|---|---|---|---|---|
| 10 | $OCH_3$ | H | $4-CH_2-$ | $-CH_2-$ | $-CH_2-$ | H | |
| 11 | $NHCH_3$ | H | $3-CH_2-$ | $-CH_2-$ | $-CH_2CH_2-$ | H | |
| 12 | $N(CH_3)_2$ | H | $3-CH_2-$ | $-CH_2-$ | $-CH_2CH_2-$ | H | 106-108 |
| 13 | $N(OCH_3)CH_3$ | H | $3-CH_2-$ | $-CH_2-$ | $-CH_2CH_2-$ | H | 110-112 |
| 14 | $N(OC_2H_5)CH_3$ | H | $3-CH_2-$ | $-CH_2-$ | $-CH_2CH_2-$ | H | |
| 15 | $N(OC_3H_5)C_2H_5$ | H | $3-CH_2-$ | $-CH_2-$ | $-CH_2CH_2-$ | H | |
| 16 | 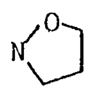 | H | $3-CH_2-$ | $-CH_2-$ | $-CH_2CH_2-$ | H | |
| 17 | 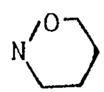 | H | $3-CH_2-$ | $-CH_2-$ | $-CH_2CH_2-$ | H | |
| 18 | $C_2H_5$ | H | $3-CH_2-$ | $-CH_2-$ | $-CH_2CH_2-$ | H | 65- 67 |
| 19 | Cyclopropyl | H | $3-CH_2-$ | $-CH_2-$ | $-CH_2CH_2-$ | H | |
| 20 | $SCH_3$ | H | $3-CH_2-$ | $-CH_2-$ | $-CH_2CH_2-$ | H | 81 |
| 21 | $OCH_3$ | H | $3-CH_2-$ | $-CH_2-$ | $-CH_2CH_2-$ | H | |
| 22 | $N(OCH_3)CH_3$ | H | $4-CH_2-$ | -CO- | $-CH_2-$ | H | 131-133 |
| 23 | $N(OCH_3)CH_3$ | H | $4-CH_2-$ | -CH(OH)- | $-CH_2-$ | H | |

(Fortsetzung)

| Nr. | $R^1$ | Y | A | X | B | $Z_n$ | Fp [°C] |
|---|---|---|---|---|---|---|---|
| 24 | $N(OCH_3)CH_3$ | H | $4-CH_2-$ | $-CO-$ | $-CH(CH_3)-$ | 3-Cl | 81 |
| 25 | $N(OCH_3)CH_3$ | H | $4-CH_2-$ | $-CH_2-$ | $-CH_2-$ | $4-OCH_3$ | 93-95 |
| 26 | $N(OCH_3)CH_3$ | H | $4-CH_2-$ | $-CH_2-$ | $-CH_2-$ | $3-CH_3$ | 106-107 |
| 27 | $N(OCH_3)CH_3$ | H | $4-CH_2-$ | $-CH_2-$ | $-CH_2-$ | $3-t-C_4H_9$ | |
| 28 | $N(OCH_3)CH_3$ | H | $4-CH_2-$ | $-CH_2-$ | $-CH_2-$ | 3-F | 82-83 |
| 29 | $N(OCH_3)CH_3$ | H | $4-CH_2-$ | $-CH_2-$ | $-CH_2-$ | $3,4-(CH_3)_2$ | |
| 30 | $N(OCH_3)CH_3$ | H | $4-CH_2-$ | $-CH_2-$ | $-CH_2-$ | $2,4-(CH_3)_2$ | |
| 31 | $N(OCH_3)CH_3$ | H | $4-CH_2-$ | $-CH_2-$ | $-CH_2-$ | $4-i-C_3H_7$ | |
| 32 | $N(OCH_3)CH_3$ | H | $4-CH_2-$ | $-CH_2-$ | $-CH_2-$ | $3,4-(OCH_3)_2$ | |
| 33 | $N(OCH_3)CH_3$ | H | $4-CH_2-$ | $-CH_2-$ | $-CH_2-$ | 4-Phenyl | |
| 34 | $N(OCH_3)CH_3$ | H | $4-CH_2-$ | $-CH_2-$ | $-CH_2-$ | 4-Phenoxy | |
| 35 | $N(OCH_3)CH_3$ | H | $4-CH_2-$ | $-CH_2-$ | $-CH_2-$ | $2,5-(CH_3)_2$ | |
| 36 | $N(OCH_3)CH_3$ | H | $4-CH_2-$ | $-CO-$ | $-CH_2-$ | 3-Br | |
| 37 | $N(OCH_3)CH_3$ | H | $4-CH_2-$ | $-CO-$ | $-CH_2-$ | 3-Cl | |
| 38 | $N(OCH_3)CH_3$ | H | $4-CH_2-$ | $-CO-$ | $-CH_2-$ | 4-Cl | |
| 39 | $N(OCH_3)CH_3$ | 3-Cl | $4-CH_2-$ | $-CO-$ | $-CH_2-$ | H | |
| 40 | $N(OCH_3)CH_3$ | $3-CH_3$ | $4-CH_2-$ | $-CH_2$ | $-CH_2-$ | H | |
| 41 | $N(OCH_3)CH_3$ | $4-CH_3$ | $3-CH_2-$ | $-CH_2-$ | $-CH_2CH_2$ | H | |

(Fortsetzung)

| Nr. | $R^1$ | Y | A | X | B | $Z_n$ | Fp [°C] |
|---|---|---|---|---|---|---|---|
| 42 | $N(OCH_3)CH_3$ | 3-Cl | $3\text{-}CH_2\text{-}$ | -CO- | $-CH_2CH_2-$ | H | |
| 43 | $N(OCH_3)CH_3$ | H | $4\text{-}(CH_2)_3$ | $-CH_2-$ | $-CH_2-$ | H | |
| 44 | $N(OCH_3)CH_3$ | H | $3\text{-}(CH_2)_3$ | $-CH_2$ | $-CH_2CH_2-$ | H | |
| 45 | $N(OCH_3)CH_3$ | H | $3\text{-}CH_2CH(CH_3)CH_2\text{-}$ | $-CH_2-$ | $-CH_2CH_2-$ | H | |
| 46 | $N(OCH_3)CH_3$ | H | $4\text{-}CH_2CH(CH_3)CH_2\text{-}$ | $-CH_2-$ | $-CH_2-$ | H | |
| 47 | $NHCH_3$ | H | $3\text{-}CH_2\text{-}$ | $-CH_2-$ | $-CH_2-$ | H | |
| 48 | $N(CH_3)_2$ | H | $3\text{-}CH_2\text{-}$ | $-CH_2-$ | $-CH_2-$ | H | 164-166 |
| 49 | $N(OCH_3)CH_3$ | H | $3\text{-}CH_2\text{-}$ | $-CH_2-$ | $-CH_2-$ | H | 89- 90 |
| 50 | $N(OC_2H_5)CH_3$ | H | $3\text{-}CH_2\text{-}$ | $-CH_2-$ | $-CH_2-$ | H | |
| 51 | $N(OC_2H_5)C_2H_5$ | H | $3\text{-}CH_2\text{-}$ | $-CH_2-$ | $-CH_2-$ | H | |
| 52 | $C_2H_5$ | H | $3\text{-}CH_2\text{-}$ | $-CH_2-$ | $-CH_2-$ | H | 82- 84 |
| 53 | $OCH_3$ | H | $3\text{-}CH_2\text{-}$ | $-CH_2-$ | $-CH_2-$ | H | 50- 51 |
| 54 | $t\text{-}C_4H_9$ | H | $3\text{-}CH_2\text{-}$ | $-CH_2-$ | $-CH_2-$ | H | |
| 55 | $SCH_3$ | H | $3\text{-}CH_2\text{-}$ | $-CH_2-$ | $-CH_2-$ | H | 104-106 |
| 56 | $N(C_2H_5)_2$ | H | $3\text{-}CH_2\text{-}$ | $-CH_2-$ | $-CH_2CH_2-$ | H | |
| 57 | $n\text{-}C_3H_7NH$ | H | $3\text{-}CH_2\text{-}$ | $-CH_2-$ | $-CH_2CH_2-$ | H | |
| 58 | N-Methyl-N-cyclohexylamino | H | $3\text{-}CH_2\text{-}$ | $-CH_2-$ | $-CH_2CH_2-$ | H | |
| 59 | 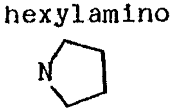 | H | $3\text{-}CH_2\text{-}$ | $-CH_2-$ | $-CH_2CH_2-$ | H | |

(Fortsetzung)

| Nr. | $R^1$ | Y | A | X | B | $Z_n$ | Fp [°C] |
|---|---|---|---|---|---|---|---|
| 60 | 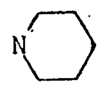 | H | 3-$CH_2$- | -$CH_2$- | -$CH_2CH_2$- | H | |
| 61 | 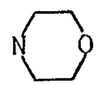 | H | 3-$CH_2$- | -$CH_2$- | -$CH_2CH_2$- | H | |
| 62 | N-Cyclopropyl-amino | H | 3-$CH_2$- | -$CH_2$- | -$CH_2CH_2$- | H | |
| 63 | $(n-C_4H_9)NC_2H_5$ | H | 3-$CH_2$- | -$CH_2$- | -$CH_2CH_2$- | H | |
| 64 | $C_2H_5$-NH | H | 3-$CH_2$- | -$CH_2$- | -$CH_2CH_2$- | H | |
| 65 | $(n-C_4H_9)$-NH | H | 3-$CH_2$- | -$CH_2$- | -$CH_2CH_2$- | H | |
| 66 | $NHCH_2CH_2CN$ | H | 3-$CH_2$- | -$CH_2$- | -$CH_2CH_2$- | H | |
| 67 | $(C_2H_5O)NH$ | H | 3-$CH_2$- | -$CH_2$- | -$CH_2CH_2$- | H | |
| 68 | $NH(CH_2CH=CH_2)$ | H | 3-$CH_2$- | -$CH_2$- | -$CH_2CH_2$- | H | |
| 69 | $(CH_3O)NH$ | H | 3-$CH_2$- | -$CH_2$- | -$CH_2CH_2$- | H | |
| 70 | $N(CH_3)C_4H_9$ | H | 3-$CH_2$- | -$CH_2$- | -$CH_2CH_2$- | H | |
| 71 | $NH(C(CH_3)_2C\equiv CH)$ | H | 3-$CH_2$- | -$CH_2$- | -$CH_2CH_2$- | H | |
| 72 | $OC_2H_5$ | H | 3-$CH_2$- | -$CH_2$- | -$CH_2CH_2$- | H | |
| 73 | O-i-$C_3H_7$ | H | 3-$CH_2$- | -$CH_2$- | -$CH_2CH_2$- | H | |
| 74 | O-sec-$C_4H_9$ | H | 3-$CH_2$- | -$CH_2$- | -$CH_2CH_2$- | H | |
| 75 | $O-CH(CH_3)C\equiv CH$ | H | 3-$CH_2$- | -$CH_2$- | -$CH_2CH_2$- | H | |

(Fortsetzung)

| Nr. | $R^1$ | Y | A | X | B | $Z_n$ | Fp [°C] |
|---|---|---|---|---|---|---|---|
| 76 | $O-CH_2CH=CH_2$ | H | $3-CH_2-$ | $-CH_2-$ | $-CH_2CH_2-$ | H | |
| 77 | Cyclohexoxy | H | $3-CH_2-$ | $-CH_2-$ | $-CH_2CH_2-$ | H | |
| 78 | $O-n-C_3H_7$ | H | $3-CH_2-$ | $-CH_2-$ | $-CH_2CH_2-$ | H | |
| 79 | $O-CH_2-C\equiv CH$ | H | $3-CH_2-$ | $-CH_2-$ | $-CH_2CH_2-$ | H | |
| 80 | $SC_2H_5$ | H | $3-CH_2-$ | $-CH_2-$ | $-CH_2CH_2-$ | H | |
| 81 | $S-t-C_4H_9$ | H | $3-CH_2-$ | $-CH_2-$ | $-CH_2CH_2-$ | H | |
| 82 | $CCl_3$ | H | $3-CH_2-$ | $-CH_2-$ | $-CH_2CH_2-$ | H | |
| 83 | $CHCl_2$ | H | $3-CH_2-$ | $-CH_2-$ | $-CH_2CH_2-$ | H | |
| 84 | $CH_2Cl$ | H | $3-CH_2-$ | $-CH_2-$ | $-CH_2CH_2-$ | H | |
| 85 | $t-C_4H_9$ | H | $3-CH_2-$ | $-CH_2-$ | $-CH_2CH_2-$ | H | |
| 86 | $CCl_2CH_3$ | H | $3-CH_2-$ | $-CH_2-$ | $-CH_2CH_2-$ | H | |
| 87 | $C(CH_3)=CH_2$ | H | $3-CH_2-$ | $-CH_2-$ | $-CH_2CH_2-$ | H | |
| 88 | $S-n-C_3H_7$ | H | $3-CH_2-$ | $-CH_2-$ | $-CH_2CH_2-$ | H | |
| 89 | $CH_2CH(CH_3)CH_3$ | H | $3-CH_2-$ | $-CH_2-$ | $-CH_2CH_2-$ | H | |
| 90 | $sec-C_5H_{11}$ | H | $3-CH_2-$ | $-CH_2-$ | $-CH_2CH_2-$ | H | |
| 91 | Cyclohexyl | H | $3-CH_2-$ | $-CH_2-$ | $-CH_2CH_2-$ | H | |
| 92 | $N(OCH_3)CH_3$ | H | $3-CH_2-$ | $-CH(OH)-$ | $-CH_2-$ | H | |
| 93 | $N(OCH_3)CH_3$ | H | $3-CH_2-$ | $-CO-$ | $-CH_2-$ | H | |

(Fortsetzung)

| Nr. | $R^1$ | Y | A | X | B | $Z_n$ | Fp [°C] |
|---|---|---|---|---|---|---|---|
| 94 | $N(OCH_3)CH_3$ | H | $3-CH_2-$ | -CO- | $-CH_2CH_2-$ | H | 99-101 |
| 95 | $N(OCH_3)CH_3$ | H | $3-CH_2-$ | -CH(OH)- | $-CH_2CH_2-$ | H | |
| 96 | $N(OCH_3)CH_3$ | H | $4-CH_2-$ | $-CH_2-$ | $-CH_2CH_2-$ | H | 133-135 |
| 97 | $N(CH_3)_2$ | H | $4-CH_2-$ | $-CH_2-$ | $-CH_2CH_2-$ | H | 144-146 |
| 98 | $C_2H_5$ | H | $4-CH_2-$ | $-CH_2-$ | $-CH_2CH_2-$ | H | 118-121 |
| 99 | $OCH_3$ | H | $4-CH_2-$ | $-CH_2-$ | $-CH_2CH_2-$ | H | |
| 100 | $SCH_3$ | H | $4-CH_2-$ | $-CH_2-$ | $-CH_2CH_2-$ | H | |
| 101 | $N(OCH_3)CH_3$ | H | $4-CH_2-$ | -CO- | $-CH_2CH_2-$ | H | |
| 102 | $N(OCH_3)CH_3$ | H | $4-CH_2-$ | -CH(OH)- | $-CH_2CH_2-$ | H | |
| 103 | $N(OCH_3)CH_3$ | H | $3-CH_2-$ | $-CH_2-$ | $-CH_2CH_2-$ | $4-OCH_3$ | |
| 104 | $N(OCH_3)CH_3$ | H | $4-CH_2-$ | -CO- | $-CH_2-$ | $3-CH_3$ | |
| 105 | $N(OCH_3)CH_3$ | H | $3-CH_2-$ | $-CH_2-$ | $-CH_2-$ | $3-CH_3$ | 90-92 |
| 106 | $N(OCH_3)CH_3$ | H | $3-CH_2-$ | $-CH_2-$ | $-CH_2-CH_2-$ | $4-CH_3$ | |
| 107 | $N(OCH_3)CH_3$ | H | $3-CH_2-$ | $-CH_2-$ | $-CH_2-$ | $2,4-(CH_3)_2$ | |
| 108 | $N(OCH_3)CH_3$ | H | $3-CH_2-$ | $-CH_2-$ | $-CH_2CH(CH_3)-$ | H | |
| 109 | $N(OCH_3)CH_3$ | $3-OCH_3$ | $4-CH_2-$ | $-CH_2-$ | $-CH_2-$ | H | |
| 110 | $N(OCH_3)CH_3$ | $3-CF_3$ | $4-CH_2-$ | $-CH_2-$ | $-CH_2-$ | H | |

(Fortsetzung)

| Nr. | $R^1$ | Y | A | X | B | $Z_n$ | Fp [°C] |
|---|---|---|---|---|---|---|---|
| 111 | $N(OCH_3)CH_3$ | H | $4-CH_2-$ | $-CH_2-$ | $-CH_2-$ | $3-CF_3$ | |
| 112 | $N(OCH_3)CH_3$ | H | $4-CH_2-$ | $-CH_2-$ | $-CH_2-$ | $3-OCHF_2$ | |
| 113 | $N(OCH_3)CH_3$ | H | $3-CH_2-$ | $-CH_2-$ | $-CH_2-$ | $3-OCH_3$ | |
| 114 | $N(OC_2H_5)CH_3$ | H | $4-CH_2-$ | -CO- | $-CH_2-$ | H | 101-104 |
| 115 | $C_2H_5$ | H | $3-CH_2-$ | -CO- | $-CH_2-CH_2-$ | H | 112-114 |
| 116 | $N(OCH_3)CH_3$ | H | $4-CH_2-$ | $-CH_2-$ | $-CH(CH_3)-$ | H | 107-108 |
| 117 | $N(OCH_3)CH_3$ | H | $3-CH_2-$ | $-CH_2-$ | $-CH(CH_3)-$ | H | 79- 81 |
| 118 | $C_2H_5$ | H | $3-CH_2-$ | $-CH_2-$ | $-CH(CH_3)-$ | H | 87- 89 |
| 119 | $N(OCH_3)CH_3$ | H | $4-CH_2-$ | $-CH_2-$ | $-C(CH_3)_2-$ | H | 136-138 |
| 120 | $N(OCH_3)CH_3$ | H | $3-CH_2-$ | $-CH_2-$ | $-C(CH_3)_2-$ | H | 116-117 |
| 121 | $C_2H_5$ | H | $3-CH_2-$ | $-CH_2-$ | $-C(CH_3)_2-$ | H | 107-109 |
| 122 | $C_2H_5$ | H | $3-CH_2-$ | $-CH_2-$ | $-CH_2-$ | $3-CH_3$ | 102-103 |
| 123 | $SCH_3$ | H | $3-CH_2-$ | $-CH_2-$ | $-CH_2-$ | $3-CH_3$ | Öl |
| 124 | $C_2H_5$ | H | $3-CH_2-$ | $-CH_2-$ | $-CH_2-$ | 3-F | 68- 71 |
| 125 | $N(OCH_3)CH_3$ | H | $3-CH_2-$ | $-CH_2-$ | $-CH_2-$ | 3-F | 72- 75 |
| 126 | $N(OCH_3)CH_3$ | H | $3-CH_2-$ | $-CH_2-$ | $-CH_2-$ | 4-Cl | Öl |
| 127 | $N(OCH_3)CH_3$ | H | $3-CH_2-$ | -CO- | $-CH_2-CH_2-$ | 3-Cl | 97-99 |
| 128 | $N(OCH_3)CH_3$ | H | $4-CH_2-$ | -CO- | $-CH_2-CH_2-$ | 3-Cl | 93-95 |
| 129 | $C_2H_5$ | H | $3-CH_2-$ | $-CH_2-$ | $-CH_2-$ | $3-OCH_3$ | 66- 68 |

Die Verbindung der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten, oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemitel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 8 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 12 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 13 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 49 werden mit 3 Gewichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer

Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 52 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 18 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 53 werden mit 2 Teilen Calciumsalz der Dodecylbenzolsulfonsäure, 8 Teilen Fettalkohol-polyglykolether, 2 Teilen Natriumsalz eines Phenol-Harstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Vorzugsweise werden die neuen Wirkstoffe nach dem Auflaufen oder während des Auflaufens der unerwünschten Pflanzen, sowohl auf Kulturflächen als auch auf unbebautem Land, ausgebracht. Sind die Wirkstoffe für die Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Bekämpfungsziel und Wachstumsstadium 0,05 bis 5 kg/ha und mehr, vorzugsweise 0,125 bis 3,0 kg/ha.

Die herbizide Wirkung von Verbindungen der Formel I wird durch Gewächshausversuche gezeigt :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Für die Nachauflaufbehandlung zieht man die Testpflanzen je nach Wuchsform erst bis zu einer Wuchshöhe von 3 bis 15 cm an und behandelt sie danach. Die für die Nachauflaufanwendung eingesetzten Sojapflanzen werden in einem mit Torfmull (peat) angereicherten Substrat angezogen, um ein günstigeres Wachstum zu gewährleisten. Zur Nachauflaufbehandlung werden entweder direkt gesäte und in den gleichen Gefäßen aufgewachsene Pflanzen ausgewählt, oder aber sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff. Sie betragen 0,25, 0,5, 1,0 oder 3,0 kg Wirkstoff/ha.

Die Versuchsgefäße werden im Gewächshaus aufgestellt, wobei für wärmeliebende Arten wärmere Bereiche (20 bis 30 °C) und für solche gemäßigter Klimate 10 bis 25 °C bevorzugt werden. Die Versuchsperiode erstreckt sich über 3 bis 4 Wochen. Während dieser Zeit werden die Pflanzen gepflegt, und ihre Reaktion auf die einzelnen Behandlungen ausgewertet. Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die Testpflanzen setzen sich aus folgenden Arten zusammen :

Abutilon theophrasti (Chinesischer Hanf), Amaranthus spp. (Fuchsschwanz), Cassia tora, Chenopodium album (Weißer Gänsefuß), Euphorbia geniculata (Südamerikanische Wolfsmilchart), Glycine max. (Soja), Ipomoea spp. (Prunkwindearten), Lamium purpureum (Rote Taubnessel), Mercurialis annua (Einjähriges Bingelkraut), Setaria italica (Kolbenhirse), Sida spinosa, Sinapis alba (weißer Senf), Solanum nigrum (Schwarzer Nachtschatten), Triticum aestivum (Weizen), Gossypium hirsutum (Baumwolle), Sesbania exaltata (Turibaum), Sorghum bicolor (Mohrenhirse), Viola spp. (Stiefmütterchen), Centaurea cyanus (Kornblume).

In diesen Tests zeigen die Verbindungen Nr. 1 und Nr. 49 mit 0,5 bzw. 0,25 kg Wirkstoff/ha eine gute Wirkung gegen unerwünschte Pflanzen, vorzugsweise dikotyle Arten, in Soja und Weizen. Die Verbindungen Nr. 26, 97 bzw. 22 bekämpfen beispielsweise mit 0,5, 1,0 bzw. 3,0 kg/ha breitblättrige unerwünschte Pflanzen selektiv. Ebenso bekämpfen die Verbindungen Nr. 18 und 52 mit 0,25 kg Wirkstoff/ha unerwünschte breitblättrige Unkräuter in Weizen.

In Anbetracht der Verträglichkeit und der Vielseitigkeit der Applikationsmethoden, können die erfindungsgemäßen Verbindungen noch in einer weiteren Zahl von Kulturpflanzen zur Beseitigung unerwünschter Pflanzen eingesetzt werden.

In Betracht kommen beispielsweise folgende Kulturen :

| Botanischer Name | Deutscher Name |
|---|---|
| Botanischer Name | Deutscher Name |
| Botanischer Name | Deutscher Name |
| Botanischer Name | Deutscher Name |
| Ananas comosus | Ananas |
| Arachis hypogaea | Erdnuß |

(Fortsetzung)

| | |
|---|---|
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gosypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst.- u. Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Theobroma cacao | Kakaobaum |
| Trifolium pratense | Rotklee |
| Triticum aestivum | Weizen |

(Fortsetzung)

| | |
|---|---|
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays (post directed) | Mais |

Zur Verbreiterung des Wirkungsspektrums und zur Erzielung synergistischer Effekte können die neuen erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

**Patentansprüche**

1. Anilinderivate der Formel

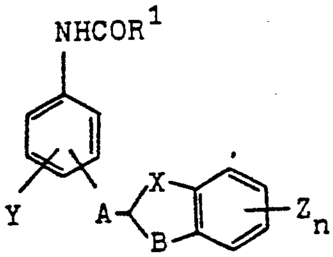

(I)

in der

$R^1$ Alkoxy, Alkenoxy, Alkinoxy, Alkylthio oder gegebenenfalls durch Halogen oder Alkoxy mit 1 bis 5 Kohlenstoffatomen substituiertes Alkyl mit jeweils bis zu 4 Kohlenstoffatomen oder Cycloalkyl mit 3 bis 6 Kohlenstoffatomen oder den Rest

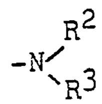

wobei $R^2$ und $R^3$ unabhängig voneinander Wasserstoff, Alkyl, Alkenyl, Alkinyl oder Alkoxy mit jeweils bis zu 4 Kohlenstoffatomen oder Cycloalkyl oder Cycloalkoxy mit 3 bis 6 Kohlenstoffatomen bedeuten oder $R^2$ und $R^3$ zusammen eine gegebenenfalls durch Methyl substituierte und gegebenenfalls Sauerstoff als Kettenglied enthaltende Alkylenkette mit bis zu 6 Kohlenstoffatomen bedeuten,

X Methylen, —CH(OH)— oder —CO—,

Y Wasserstoff, Halogen, Methyl, Methoxy oder Trifluormethyl,

A eine gegebenenfalls durch Alkyl mit 1 bis 5 Kohlenstoffatomen substituierte Alkylenkette mit 1 bis 5 Kohlenstoffatomen,

B gegebenenfalls durch Alkyl mit 1 bis 3 Kohlenstoffatomen substituiertes Methylen oder Dimethylen,

Z Wasserstoff, Halogen, Alkyl, Alkoxy, Halogenalkyl oder Halogenalkoxy mit jeweils bis zu 6 Kohlenstoffatomen, Phenyl oder Phenoxy und

n die Zahlen 1, 2 oder 3 bedeuten.

2. Anilinderivate der Formel I gemäß Anspruch 1, dadurch gekennzeichnet, daß $R^1$ den Rest

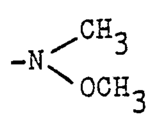

Y Wasserstoff oder Halogen, A Methylen in para-Stellung zu der Gruppe —NH—CO—$R^1$, X Methylen, B Methylen, Z Halogen, Wasserstoff oder Alkyl mit bis zu 4 Kohlenstoffatomenund n 1 bedeuten.

3. Anilinderivate der Formel I gemäß Anspruch 1, dadurch gekennzeichnet, daß $R^1$ den Rest

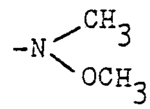

Y Wasserstoff oder Chlor, A Methylen in meta-Stellung zu der Gruppe —NH—CO—$R^1$, X Methylen, B Dimethylen, Z Chlor, Wasserstoff oder Methyl und n 1 bedeuten.

4. N-[4-(Indan-2-yl-methyl)-phenyl]-N'-methoxy-N'-methylharnstoff.

5. Verfahren zur Herstellung der Anilinderivate der Formel I gemäß Anspruch 1, dadurch gekennzeichnet, daß man ein Amin der Formel

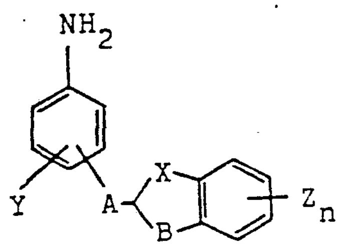

(II)

in der A, Y, X, B, Z und n die im Anspruch 1 genannten Bedeutungen haben, mit einer Verbindung der Formel

$$R^1—CO—X \qquad (III)$$

in der $R^1$ die im Anspruch 1 angegebenen Bedeutungen hat und X eine Abgangsgruppe oder den Rest $R^1$—CO—O— bedeutet, in Gegenwart eines inerten organischen Lösungsmittels und eines Säureakzeptors bei einer Temperatur im Bereich zwischen 20 und 80 °C umsetzt.

6. Herbizid, enthaltend ein Anilinderivat der Formel I gemäß Anspruch 1.

7. Herbizid, enthaltend inerte Zusatzstoffe und ein Anilinderivat der Formel I gemäß Anspruch 1.

8. Verfahren zur Bekämpfung unerwünschten Pflanzenwuchses, dadurch gekennzeichnet, daß man eine herbizid wirksame Menge eines Anilinderivats der Formel I gemäß Anspruch 1 auf die Pflanzen und/oder ihren Standort einwirken läßt.

9. Amine der Formel

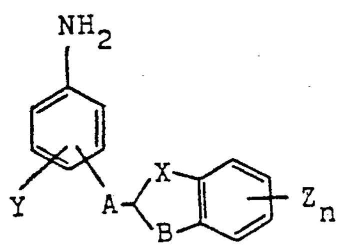

(II)

X Methylen, —CH(OH)— oder —CO—,

Y Wasserstoff, Halogen, Methyl, Methoxy oder Trifluormethyl,

A eine gegebenenfalls durch Alkyl mit 1 bis 5 Kohlenstoffatomen substituierte Alkylenkette mit 1 bis 5 Kohlenstoffatomen,

B gegebenenfalls durch Alkyl mit 1 bis 3 Kohlenstoffatomen substituiertes Methylen oder Dimethylen,

Z Wasserstoff, Halogen, Alkyl, Alkoxy, Halogenalkyl oder Halogenalkoxy mit jeweils bis zu 6 Kohlenstoffatomen, Phenyl oder Phenoxy und

n die Zahlen 1, 2 oder 3 bedeuten.

10. Verwendung von Aminen der Formel II gemäß Anspruch 9 zur Herstellung von Anilinderivaten der Formel I gemäß Anspruch 1.

## Claims

1. An aniline derivative of the formula

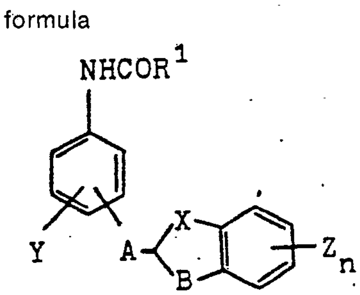

(I)

where

$R^1$ is alkoxy, alkenoxy, alkynoxy, alkylthio or unsubstituted, halogen-substituted or $C_1$-$C_5$-alkoxy-substituted alkyl, each of not more than 4 carbon atoms, cycloalkyl of 3 to 6 carbon atoms or

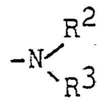

where $R^2$ and $R^3$ independently of one another are hydrogen, alkyl, alkenyl, alkynyl or alkoxy, each of not more than 4 carbon atoms, or cycloalkyl or cycloalkoxy of 3 to 6 carbon or $R^2$ and $R^3$ together form an alkylene chain of not more than 6 carbon atoms which is unsubstituted or substituted by methyl and may or may not contain oxygen as a chain member,

X is methylene, —CH(OH)— or —CO—,

Y is hydrogen, halogen, methyl, methoxy or trifluoromethyl,

A is an alkylene chain of 1 to 5 carbon atoms which is unsubstituted or substituted by alkyl of 1 to 5 carbon atoms,

B is dimethylene or methylene which is unsubstituted or substituted by alkyl of 1 to 3 carbon atoms.

Z is hydrogen, halogen, alkyl, alkoxy, haloalkyl or haloalkoxy, each of not more than 6 carbon atoms, or phenyl or phenoxy, and

n is 1, 2 or 3.

2. An aniline derivative of the formula I as claimed in claim 1, where $R^1$ is

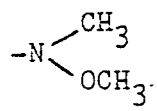

Y is hydrogen or halogen, A is methylene para to —NH—CO—$R^1$, X is methylene, B is methylene, Z is halogen, hydrogen or alkyl of not more than 4 carbon atoms, and n is 1.

3. An aniline derivative of the formula I as claimed in claim 1, where $R^1$ is

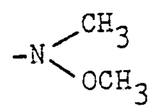

Y is hydrogen or chlorine, A is methylene meta to —NH—CO—$R^1$, X is methylene, B is dimethylene, Z is chlorine, hydrogen or methyl, and n is 1.

4. N-[4-(Indan-2-yl-methyl)-phenyl]-N'-methoxy-N'-methylurea.

5. A process for the preparation of an aniline derivative of the formula I as claimed in claim 1, wherein an amine of the formula

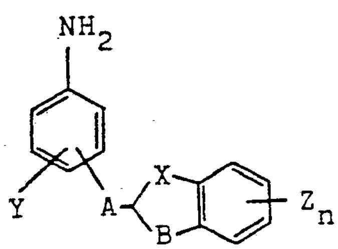

(II)

where A, Y, X, B, Z and n have the meanings given in claim 1, is reacted with a compound of the formula

$$R^1—CO—X \quad (III),$$

where $R^1$ has the meanings given in claim 1 and X is a leaving group or $R^1$—CO—O—, in the presence of an inert organic solvent and an acid acceptor at a temperature of from 20 to 80 °C.

6. A herbicide containing an aniline derivative of the formula I as claimed in claim 1.

7. A herbicide containing inert additives and an aniline derivative of the formula I as claimed in claim 1.

8. A process for combating the growth of unwanted plants, wherein a herbicidally effective amount of an aniline derivative of the formula I as claimed in claim 1 is allowed to act on the plants and/or their location.

9. An amine of the formula

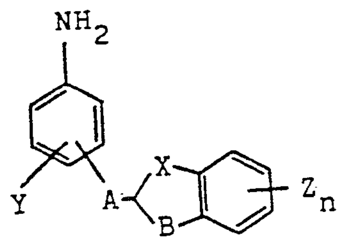

(II)

where

X is methylene, —CH(OH)— or —CO—,

Y is hydrogen, halogen, methyl, methoxy or trifluoromethyl,

A is an alkylene chain of 1 to 5 carbon atoms which is unsubstituted or substituted by alkyl of 1 to 5 carbon atoms,

B is dimethylene or methylene which is unsubstituted or substituted by alkyl of 1 to 3 carbon atoms

Z is hydrogen, halogen, alkyl, alkoxy, haloalkyl or haloalkoxy, each of not more than 6 carbon atoms, or phenyl or phenoxy, and

n is 1, 2 or 3.

10. The use of an amine of the formula II as claimed in claim 9 for the preparation of an aniline derivative of the formula I as claimed in claim 1.

**Revendications**

1. Dérivés de l'aniline de la formule

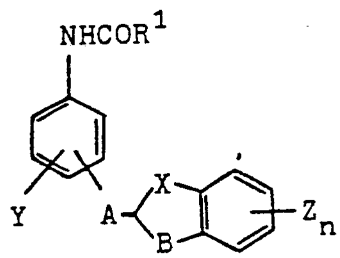

(I)

dans laquelle

$R^1$ désigne un groupe alcoxy, alcénoxy, alcynoxy ou alkylthio ou un radical alkyle en $C_1$ à $C_4$ éventuellement halo- ou alcoxy (en $C_1$ à $C_5$) substitué ou un groupe cycloalkyle en $C_3$ à $C_6$ ou un groupe

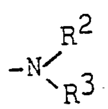

où $R^2$ et $R^3$, indépendamment l'un de l'autre, peuvent désigner chacun un atome d'hydrogène ou un radical alkyle, alcényle, alcynyle ou alcoxy comportant jusqu'à quatre atomes de carbone ou un groupe cycloalkyle ou cycloalcoxy en $C_3$ à $C_6$ ou former ensemble une chaîne alkylène comportant jusqu'à six atomes de carbone, éventuellement méthylsubstituée ou comprenant de l'oxygène comme maillon de la chaîne,

X = méthylène, —CH(OH)— ou —CO—,

Y = H, halogène, méthyle, méthoxy ou trifluorométhyle,

A représente une chaîne alkylène en $C_1$ à $C_5$ éventuellement alkyl (en $C_1$ à $C_5$)-substituée,

B un groupe méthylène ou di-méthylène éventuellement alkyl (en $C_1$ à $C_3$)-substitué,

Z = H, halogène, alkyle, alcoxy, halo-alkyle ou haloalcoxy comprenant jusqu'à six atomes de carbone, phényle ou phénoxy et

n est un nombre valant 1, 2 ou 3.

2. Dérivés de l'aniline de la formule I selon la revendication 1, caractérisés en ce que

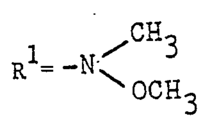

Y = hydrogène ou halogène, A = méthylène en position para par rapport au groupe —NH—CO—$R^1$, X = méthylène, B = méthylène, Z = halogène, hydrogène ou alkyle en $C_1$ à $C_4$ et n = 1.

3. Dérivés de l'aniline de la formule I selon la revendication 1, caractérisés en ce que

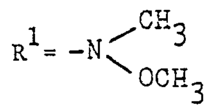

Y = H ou Cl, A = méthylène en position méta par rapport au groupe —NH—CO—$R^1$, X = méthylène, B = diméthylène, Z = Cl, H ou méthyle et n = 1.

4. La N-[4-(indan-2-yl-méthyl)-phényl]-N'-méthoxy-N'-méthyl-urée.

5. Procédé de préparation des dérivés de l'aniline de la formule I selon la revendication 1, caractérisé en ce que l'on fait réagir une amine de la formule

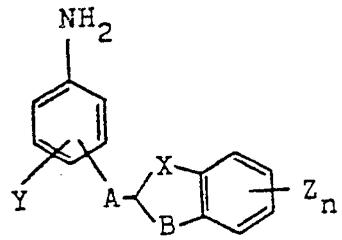

(II)

dans laquelle A, Y, X, B, Z et n possèdent les significations définies dans la revendication 1, dans la gamme de températures de 20 à 80 °C, dans un solvant organique inerte et en présence d'un fixateur d'acide, avec un composé de la formule

$R^1$—CO—X (III),

dans laquelle $R^1$ possède les significations définies dans la revendication 1 et X désigne un groupe clivable ou un groupe $R^1$—CO—O—.

6. Composition herbicide, contenant un dérivé de l'aniline de la formule I selon la revendication 1.

7. Composition herbicide, contenant un dérivé de l'aniline de la formule I selon la revendication 1 dans des additifs inertes.

8. Procédé de lutte contre le développement de plantes indésirables, caractérisé en ce que l'on fait agir sur les plantes et(ou) leur biotope une quantité efficace du point de vue herbicide d'un dérivé de l'aniline de la formule I selon la revendication 1.

9. Amines de la formule

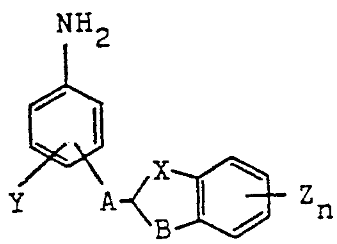

(II)

dans laquelle

X = méthylène, —CH(OH)— ou —CO—,

Y = hydrogène, halogène, méthyle, méthoxy ou trifluorométhyle,

A = une chaîne alkylène en $C_1$ à $C_5$ éventuellement alkyl (en $C_1$ à $C_5$)-substituée,

B = méthylène ou di-méthylène éventuellement alkyl (en $C_1$ à $C_3$) substitué,

Z = hydrogène, halogène, alkyle, alcoxy, halo-alkyle ou halo-alcoxy comportant jusqu'à six atomes de carbone ou phényle ou phénoxy et

n = 1, 2 ou 3.

10. Utilisation d'amines de la formule II selon la revendication 9 pour la préparation de dérivés d'aniline de la formule I selon la revendication 1.